# EUROPEAN PATENT APPLICATION

(11) **EP 2 138 511 A1**
(43) Date of publication of application: **30.12.2009**
(21) Application number: 08011736.9
(22) Date of filing: 27.06.2008
(51) Int. Cl.: C07K 14/71, C07K 16/28, C07K 16/32, C12Q 1/68, G01N 33/574, A61P 35/04

(54) **HER3 as a determinant for the prognosis of melanoma**

(71) Applicant: Max-Planck-Gesellschaft zur Förderung der Wissenschaften e.V., 80539 München (DE)
(72) Inventor: The designation of the inventor has not yet been filed
(74) Representative: Weiss, Wolfgang

(57) **Abstract**

The present invention relates to an inhibitor of HER3 for the treatment of melanoma, pharmaceutical compositions comprising such an inhibitor and a method for the diagnosis or prognosis of melanoma.

## Description

The present invention relates to an inhibitor of HER3 for the treatment of melanoma, pharmaceutical compositions comprising such an inhibitor and a method for the diagnosis or prognosis of melanoma.

Melanoma is a common type of skin cancer, which develops from the malignant transformation of melanocytes, and accounts for 80% of deaths arising from skin cancer (1). The underlying molecular mechanisms of melanocyte transformation have been studied extensively in the past (2, 3).

The human EGF receptor (HER) family of receptor tyrosine kinases regulates a large variety of biological processes including cell proliferation, -migration, -invasion and -survival (5). The family consists of four members: EGFR (HER1), HER2 (neu or ErbB2), HER3 (ErbB3) and HER4 (ErbB4). To date, eleven ligands have been reported including epidermal growth factor (EGF), heparin-binding EGF-like growth factor (HB-EGF), transforming growth factor α (TGFα), amphiregulin (AR), epiregulin, betacellulin and the heregulins. These ligands bind directly to their cognate receptors, which leads to the formation of receptor homo- or heterodimers that trigger the activation of multiple signaling pathways (6). Dysregulation of members of the HER-family either by activating mutations, receptor over expression or aberrant ligand release leads to the development of a variety of human tumors (7). HER3 is over expressed in breast-, ovarian- and lung cancer and this genetic feature has been correlated with poor prognosis (8 - 10). Upon activation by heregulins, HER3 dimerizes with HER2 and EGFR to form potent oncogenic receptor heterodimers (11 - 13). Within this complex, HER3 preferentially recruits PI3-kinase to its cytoplasmic docking sites thereby regulating cell proliferation and -survival (14, 15). So far it was assumed that HER3 is kinase-inactive due to apparently aberrant sequence characteristics in its kinase domain and that it requires heterodimerization with a kinase-intact member of the HER-family in order to initiate signaling events (16). Consistent with this, it was shown that HER2 requires HER3 to drive breast tumor cell proliferation (17). However, recent findings of Htuhn van der Horst and coworkers showed that HER3 is able to phosphorylate Pyk2 which results in the activation of the MAPK pathway in human glioma cells (18). Furthermore, monoclonal antibodies specific for HER3 can inhibit the proliferation and migration of cancer cell lines (19). Interestingly, it was shown recently that cancer cells escape HER-family inhibitor therapy by up-regulation of HER3 signaling (20) and that HER3 inhibition abrogates HER2-driven tamoxifen resistance in breast cancer cells (21). Moreover, resistance to Gefitinib (Iressa) therapy, an EGFR small molecule inhibitor, was shown to be connected to HER3 signal activation (22).

The discovery of animal oncogenes that are derived from genes encoding receptor tyrosine kinases has led over the past twenty years to the development of several targeted therapeutics with the HER2 monoclonal antibody Trastuzumab being the first in clinical application for the treatment of metastatic breast carcinoma with HER2 gene amplification. However, in spite of further advances in the development of side effect-poor therapies for major malignancies such as breast cancer, there is still a great unmet need for better, more effective therapies for other cancer types. Melanoma is a highly aggressive skin cancer and current therapies only show limited efficacy in patients with late stage disease (4, 34). So far it is known that the Ras-Raf-MAPK and the PI3K-AKT pathways are frequently activated in malignant melanoma and that this contributes to tumor progression (35).

To date no drugs are available that significantly prolong patient survival once melanoma progresses to the metastatic state (4). Thus, there is an urgent need for novel therapeutic agents and prognostic markers in the treatment of melanoma patients.

Thus, the present invention relates in one aspect to an inhibitor of HER3 for the treatment of melanoma, preferably refractory melanoma.

According to the present invention the term "inhibitor of HER3" comprises every compound which may inhibit the transcription or translation of HER3 or which may act on the protein level, i.e. inhibit the HER3 activity, in particular HER3 mediated signal transduction. The activity of HER3 may also be inhibited on the gene level. Inhibition on the gene level may comprise a partial or complete gene inactivation, e.g. by gene disruption.

The terms "inhibitor of HER3" and "HER3 inhibitor" may be used interchangeable herein.

In a preferred embodiment the HER3 inhibitor is selected from the group consisting of nucleic acids, in particular small interfering RNA (siRNA), antisense oligonucleotides or ribozymes, peptidic compounds, in particular antibodies or antibody fragments and small organic non-peptidic molecules, i.e. molecules having a low molecular weight, and combinations thereof. Preferably, the HER3 inhibitor according to the invention is an anti-HER3-antibody or HER3 specific siRNA.

In the context of the present invention, the term"antibody"covers monoclonal antibodies, polyclonal antibodies, multispecific antibodies (e.g. bispecific antibodies) formed from at least two antibodies and antibody fragments as long as they exhibit the desired activity.

An especially preferred anti-HER3 antibody is an antibody directed against the extracellular domain of HER3. Said anti-HER3 antibody is preferably monoclonal.

A monoclonal antibody may be obtained by the hybridoma method as described by Kohler et al. (Nature 256 (1975), 495) or by recombinant DNA methods (cf. e.g. U. S. Patent 4,816, 567). Monoclonal antibodies may also be isolated from phage antibody libraries using techniques which are known to the person skilled in the art. The antibody may be an IgM, IgG, e. g.IgG1, IgG2, IgG3 or IgG4.

Antibody fragments comprise a portion of an antibody, generally the antigen binding or variable region of the intact antibody. Examples of antibody fragments include Fab, Fab', F (ab') 2 and Fv fragments, diabodies, single chain antibody molecules and multispecific antibody fragments.

Particularly, the antibody may be a recombinant antibody or antibody fragment, more particularly selected from chimeric antibodies or fragments thereof and diabodies. For therapeutic purposes, particularly for the treatment of humans, the administration of chimeric antibodies, humanized antibodies or human antibodies is especially preferred.

The anti-HER3 antibodies used according to the invention may be coupled to a labelling group, particularly for diagnostic applications. Examples for suitable labelling groups such as radioactive groups, fluorescent groups or other labelling groups are known in the art. Further, particularly for therapeutic applications, the antibody may be coupled to an effector group, which may be effective in the treatment of melanoma, e.g. a cytotoxic group such as a radioactive group, a toxin or another effector group as known in the art.

SiRNA used according to the invention is a double-strand of RNA and/or nucleotide analogues with 3' overhangs on at least one end, preferably either ends. Each RNA strand of the double-strand has a 5' phosphate group and a 3' hydroxyl group. Preferably, each RNA strand of the double strand is 20 to 25 nucleotides long, more preferably 20 to 23 nucleotides and most preferably 21 nucleotides. The 3' overhang on the end of a RNA strand is preferably 2 nucleotides long. In a particular preferred embodiment the siRNA double-strand consists of two 21 nucleotides long RNA strands each having a 2 nucleotides long 3' overhang. Methods for obtaining siRNA molecules are known to the person skilled in the art.

SiRNA molecules may be applied to a target cell by any technique which is known to the person skilled in the art, such as transfection of exogenous siRNA or of an appropriate vector, e.g. viral or non-viral, producing a single transcript, which can be processed into a functional siRNA.

Ribozymes may be selected from RNA molecules and nucleic acid analogues. Suitable anti-sense molecules may be selected from DNA molecules, RNA molecules and nucleic acid analogues.

In an preferred embodiment of the invention the HER3 inhibitor is an inhibitor of HER3 expression, which preferably acts by down-regulation or knock-down of HER3. Down-regulation or knock-down of HER3 results preferably in an at least partial disappearance of HER3 molecules from the cell surface. In a particular preferred embodiment HER3 expression is inhibited by the use of specific siRNAs. Especially preferred HER3 specific siRNAs are selected from the group consisting of
5' GGCUAUGUCCUCGUGGCCAtt 3' (sense1),
5' UGGCCACGAGGACAUAGCCtg 3' (antisense1),
5' GGCAGUGUGUCCUGGGACUtt 3' (sense2),
5' AGUCCCAGGACACACUGCCtg 3' (antisense2) or a combination thereof.

HER3 down-regulation or knock-down may lead to impaired AKT activation, reduced Cyclin B1 levels and/or decreased Rb phosphorylation. Preferably, HER2 protein levels are not altered in HER3 down-regulated cells.

In a particular preferred embodiment, HER3 down-regulation or knock-down inhibits melanoma cell proliferation, migration and/or invasion.

In a further preferred embodiment the HER3 inhibitor is an inhibitor of Heregulin-induced HER3 activation. The HER3 inhibitor may in particular inhibit Heregulin-induced HER3 phosphorylation leading to receptor internalization or degradation. The inhibitor used according to invention may influence the binding of Heregulin to HER3, particularly by decreasing the binding of Heregulin to HER3. In a particular preferred embodiment, the HER3 inhibitor, preferably an anti-HER3 antibody, is used to inhibit Heregulin-induced migration and invasion of melanoma cells.

In yet a further embodiment the HER3 inhibitor is characterized in that binding of the inhibitor to HER3 reduces HER3 mediated signal transduction. HER3 mediated signal transduction is preferably reduced by a down-regulation of HER3 resulting in an at least partial disappearance of HER3 molecules from the cell surface or by a stabilization of HER3 on the cell surface in a substantially inactive form, i.e. a form which exhibits a lower signal transduction compared to the non-stabilized form. An anti-HER3-antibody is a preferred embodiment of a HER3 inhibitor reducing HER3 mediated signal transduction.

According to the invention the term "melanoma" denotes preferably malignant tumours of melanocytes which are found predominantly in skin but also in the bowel and the eye. Malignant melanoma is due to uncontrolled growth of pigment cells, i.e. melanocytes. According to an especially preferred embodiment of the invention melanoma is primary melanoma or metastases and in particular refractory melanoma or therapy resistant melanoma. Metastases, in particular micrometastases, are often not accessible by surgery and need pharmacological or/and irradiation treatment.

According to the invention, the terms "refractory melanoma" or "therapy resistant melanoma" include the inability of hyperproliferative cells/cancer cells, in particular melanoma cells, to respond to therapy, i.e. the inability of a therapeutic treatment to reduce or suppress cell proliferation or/and to induce cell death in hyperproliferative cells/cancer cells. A serious form of therapy resistance included herein is multi-drug resistance. "Therapy resistant" or "refractory melanoma" also includes resistance of melanoma against a monotherapy, in particular with a cytostatic, cytotoxic or/and chemotherapeutic agent such as an apoptosis-inducing agent or by irradiation therapy. "Therapy resistant" or "refractory melanoma" also includes resistance to a combination treatment of at least two selected from cytostatic, cytotoxic and chemotherapeutic agents and irradiation. According to the invention at least partially therapy resistant melanoma is also included. In the context of the present invention "at least partially therapy resistant" includes temporal development of therapy resistance from full responsiveness to an anti-cancer treatment regimen to complete resistance against this treatment.

The melanoma to be treated according to the invention is preferably selected from the group consisting of malignant melanoma, clear cell sarcoma (melanoma of soft parts), mucosal melanoma and/or uveal melanoma. Common types of melanoma in the skin comprise superficial spreading melanoma (SSM), nodular melanoma, acral lentiginous melanoma and lentigo melanoma.

According to another embodiment the HER3 inhibitor used according to the invention is an inhibitor of melanoma cell proliferation, migration and/or invasion, i.e. a reduction of HER3, in particular of HER3 expression or HER3 activity, results in reduced cell proliferation, migration and/or invasion. Tumor cell migration and invasion are important prerequisites for metastasis. Preferably, melanoma cell migration and/or invasion is blocked by inhibiting HER3 signaling via the P13K pathway by a HER3 inhibitor, in particular an anti-HER3 antibody. In contrast, a monoclonal antibody specific for HER2 does not affect melanoma cell invasion. As already outlined above, according to another preferred embodiment of the invention, melanoma cell proliferation, migration and/or invasion is inhibited by HER3 down-regulation or knock-down.

Preferably, the HER3 inhibitor is an enhancer of apoptosis. Moreover, the HER 3 inhibitor preferably sensitizes the melanoma for treatment with a further active agent or radiation therapy, i.e. acts synergistically with the active agent or radiation therapy. Such an active agent may be selected from the group consisting of chemotherapeutic agents, immunotherapeutic agents and/or adjuvant agents. Preferably, the agent is a chemotherapeutic agent.

According to an especially preferred embodiment HER3 knock-down or down-regulation sensitizes melanoma cells to apoptosis.

A further aspect of the present invention relates to a pharmaceutical composition comprising an inhibitor of HER3 as defined herein.

The pharmaceutical composition may be formulated by mixing the active agent, i.e. the HER3 inhibitor with physiologically acceptable carriers, diluents, excipients and/or adjuvants, e.g. in the form of lyophilized formulations, aqueous solutions, ointments, emulsions, suspensions, dispersions or solid preparations such as tablets, powders, dragees or capsules as described in Remington's Pharmaceutical Sciences.

The composition may be administered by injection, orally, topically, rectally, intranasally or by any other suitable means. The composition may be administered by one dose per day or may be divided up into several doses. The composition may be also administered in a continuous way, e.g. by infusion.

Furthermore, one may administer the pharmaceutical composition of the present invention in a targeted drug delivery system, for example in a liposome coated with a tumor-specific antibody. The liposomes will be targeted to and taken up selectively by the tumor.

The effective amount of the active agent, i.e. the HER3 inhibitor in the composition may be determined by the skilled person without any undue burden depending on the kind of active agent and the kind of melanoma to be treated. For example, about 1 µg/kg to 15 mg/kg of a HER3 inhibitor, such as an anti-HER3 antibody, may be administered to a human patient, e.g. by one or more separate administrations or by continuous infusion. A typical daily dosage might range from about 1 µg/kg to about 100 mg/kg or more, depending on the factors such as age, gender, weight, tumor thickness and condition of the person to be treated etc. For repeated administrations over several days or longer, depending on the condition to be treated, the treatment is sustained until a desired suppression of disease symptoms occurs.

Radiation treatment regimens are known by a person skilled in the art.

In a preferred embodiment the pharmaceutical composition is to be administered in combination with further cytostatic, cytotoxic, chemotherapeutic and/or immunotherapeutic agents and/or before, during and/or after surgical removal of the melanoma and/or radiation therapy.

Thus, in a further preferred embodiment the pharmaceutical composition according to the invention comprises at least one further active agent, which is preferably active in the treatment of melanoma such as a further chemotherapeutic and/or immunotherapeutic agent.

According to the invention chemotherapeutic agents may preferably be selected from the group consisting of Dacarbazine (DTIC), Herceptin, Omnitarg, Lapatinib, Temozolomide and/or Procarbazine. As shown by the present invention, down-regulation of HER3 synergistically enhances Dacarbazine-induced apoptosis. Thus, Dacarbazine is an especially preferred chemotherapeutic agent.

Immunotherapeutic agents comprise for example interleukin-2 (IL-2) or interferon (IFN).

According to another preferred embodiment, a pharmaceutical composition as defined in the present invention may be administered as part of a combination therapy. In the context of the present invention, "combination therapy" refers to the simultaneous administration of two or more active agents as defined above, wherein at least one of these agents is a HER3 inhibitor according to the present invention. The two or more active agents may be administered simultaneously in one single pharmaceutical composition or more than one pharmaceutical composition, wherein each composition comprises at least one active agent. However, the term "combination therapy" refers also to other types of therapy such as radiation which are used at the same time.

According to the present invention it is demonstrated that HER3 is expressed in malignant melanoma and metastases at elevated levels. It was found that, high HER3 expression correlates with cell proliferation, tumor progression and/or reduced patient survival. HER3 is undetectable in primary melanocytes. Thus, HER3 expression may serve as a prognostic marker for melanoma.

Accordingly, one aspect of the present invention relates to the use of HER3 as prognostic marker for melanoma.

The subject in need of treatment may be any animal which may suffer from melanoma, in particular a mammal, more particularly a human being.

A further aspect of the present invention relates to an in vitro method for the diagnosis and/or prognosis of melanoma comprising
(a) detecting HER3 levels in cells to be analysed and
(b) correlating the detected HER3 levels with proliferation, tumor progression and/or patient survival.

In general, detection of HER3 levels can be done by any method which is known by the person skilled in the art. In particular, the HER3 levels or HER3 expression may be determined on the nucleic acid level or on the protein level according to standard methods, e.g. using a gene array or immunochemical, i.e. immunohistochemical methods. An overexpression of HER3 is determined by comparing the HER3 expression in the.sample to be analysed with the HER3 expression in control samples, e.g. samples from healthy subjects or with standard values. The cells to be analysed may be a tissue sample, e.g. a biopsy.

Commonly, up-regulated HER3 levels indicate melanoma.

The in vitro method may be combined with other methods for the diagnosis or prognosis of melanoma which are known in the art such as dermatoscopic exam, biopsy, X-rays, ultrasound, lactate dehydrogenase (LDH) testing and/or photoacoustic detection.

### Figure legends

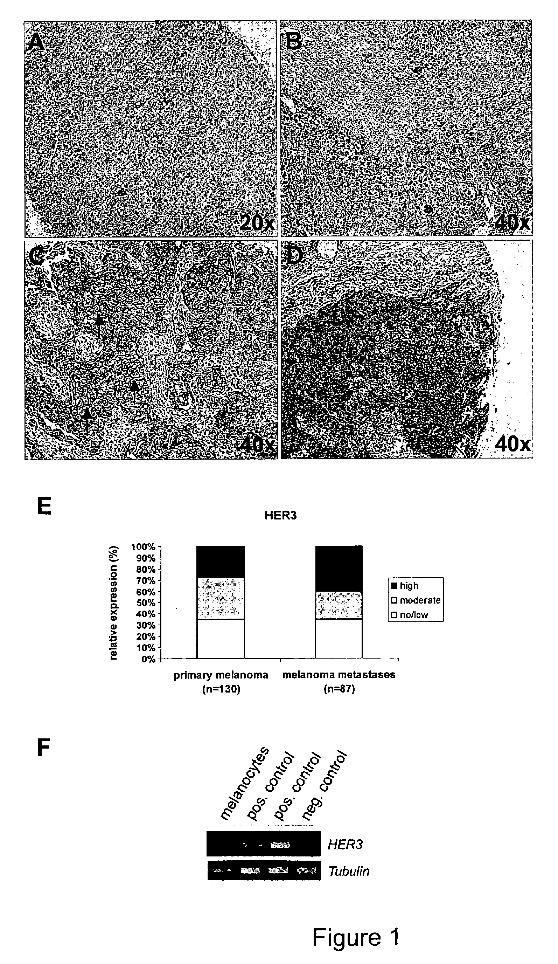

### Fig. 1 HER3 expression in primary melanoma and metastases.

Immunohistochemical staining of HER3 in primary melanoma and metastases. **A** Low HER3 expression (20x), **B** Moderate HER3 expression (40x) and **C** High HER3 expression (40x) in primary melanoma. HER3 immunoreactivity is accentuated at the cell membrane (black arrows). D High HER3 expression in melanoma metastases (40x). **E** HER3 expression frequencies in primary melanoma and metastases. **F** Absence of HER3 expression in primary melanocytes. HER3 expressing and nonexpressing melanoma cell lines served as positive and negative controls, respectively. Tubulin served as a loading control.

### Fig. 2 HER3 protein expression confers poor prognosis in melanoma patients.

**A** HER3 expression increases during melanoma progression. 20 primary tumors with matching melanoma metastases where evaluated based on their GIS score for HER3. **B** Kaplan-Meier analysis of tumor-specific survival according to HER3 expression levels (p = 0,014).

### Fig. 3 HER3 knock-down inhibits melanoma cell proliferation

**A** *HER3* knock-down in Colo 829 and MM-358 melanoma cells. Western blots are shown for HER3 and Tubulin. **B** *HER3* knock-down inhibits proliferation of Colo 829 and MM-358 melanoma cells. Bright field pictures were taken using a Zwiss Axiovert 300 microscope at a 10x magnification and growth curves were done by counting cells at the indicated time points using an automated cell counter. The data are shown as mean ± SDM. **C** *HER3* knock-down impairs AKT activity upon heregulin β1 stimulation and induces a growth arrest in Colo 829 and MM-358 melanoma cells. Western blots are shown for, p-AKT, AKT, p27, p-Rb, Cyclin B1, p-ERK1/2, p-mTOR and Tubulin.

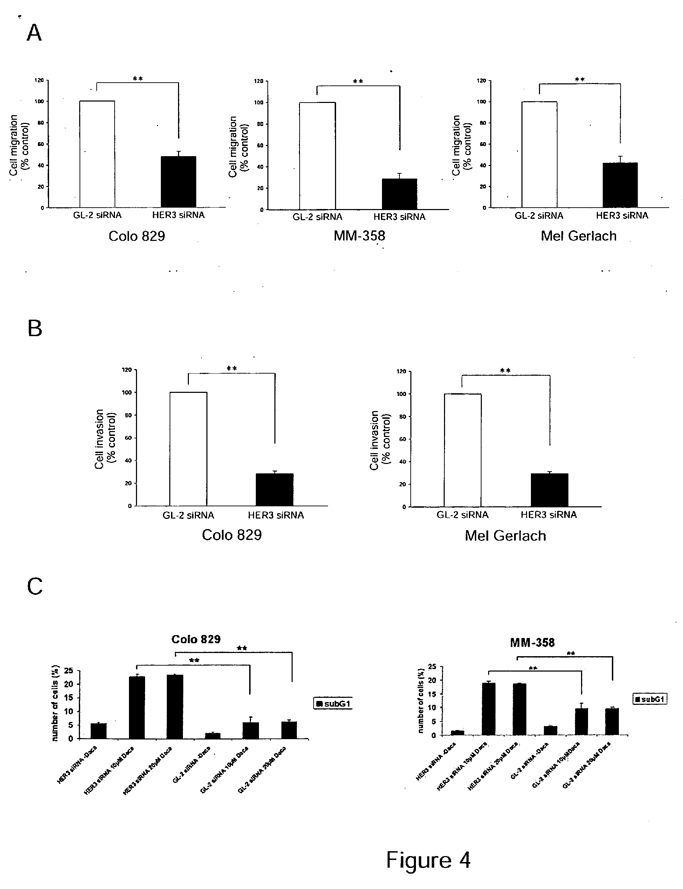

### Fig. 4 HER3 knock-down inhibits melanoma cell migration and invasion and induces apoptosis in response to chemotherapeutic drugs.

**A** *HER3* knock-down inhibits migration of Colo 829, MM-358 and Mel Gerlach melanoma cells. For quantification, pictures of migrated cells were taken on a Zeiss Axiovert 300 microscope and cells were counted in at least 10 random fields. The data are shown as mean ± SDM. **B** *HER3* knock-down inhibits invasion of Colo 829 and Mel Gerlach melanoma cells. Quantification of invaded cells was done as described in A. **C** Induction of apoptosis in Colo 829 and MM-358 *HER3* knock-down cells upon treatment with increasing amounts (10µM and 20µM) of Dacarbazine. Data are shown as mean ± SDM.

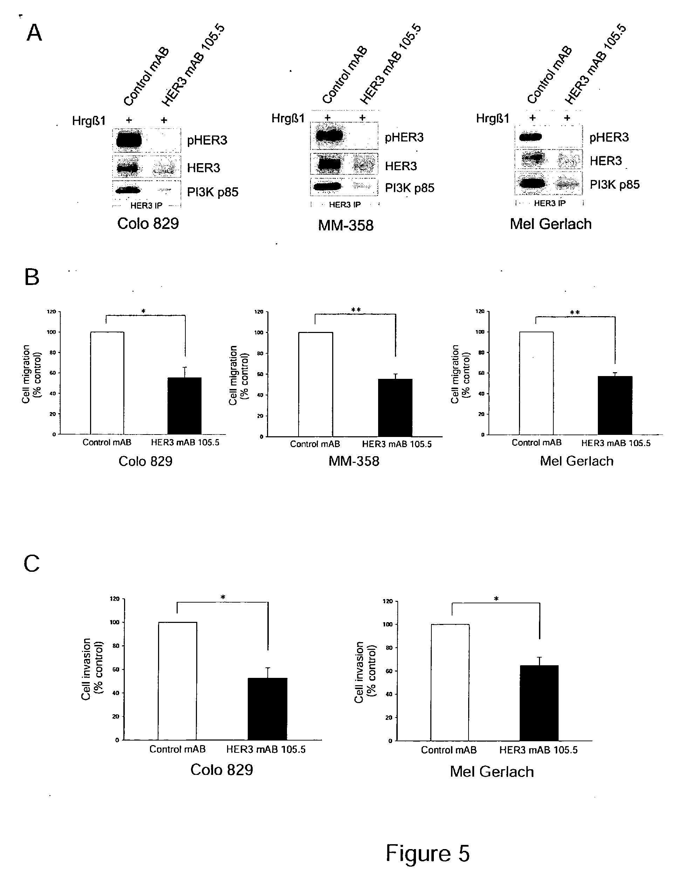

### Fig. 5 An Anti-HER3 monoclonal antibody (cl. 105.5) inhibits HER3 activation and blocks melanoma cell migration and invasion.

**A** Anti-HER3 antibody treatment blocks HER3 activation, its association with p85 and leads to receptor internalization or degradation. Colo 829, MM358 and Mel Gerlach melanoma cells were either incubated with the HER3 blocking antibody (cl. 105.5) or an isotype control antibody, stimulated with Hrgβ1, lysed and equal amounts of protein was subjected to immunoprecipitations using a specific HER3 antibody. Western blots for p-HER3 (Y1289), HER3 and p85 are shown. **B** An Anti-HER3 antibody (cl. 105.5) blocks melanoma cell migration *in vitro.* The cells were either incubated with the Anti-HER3 antibody or an isotype control antibody. The migration assay was done in a modified boyden chamber. Conditioned NIH3T3 medium containing 100ng/ml Heregulin β1 was used as a chemoattractant. The quantification was done as described in Figure 4. The data are shown as mean ± SDM. **C** An Anti-HER3 antibody (cl. 105.5) blocks melanoma cell invasion *in vitro.* The assay was done as in B using growth factor-reduced matrigel in a modified boyden chamber.

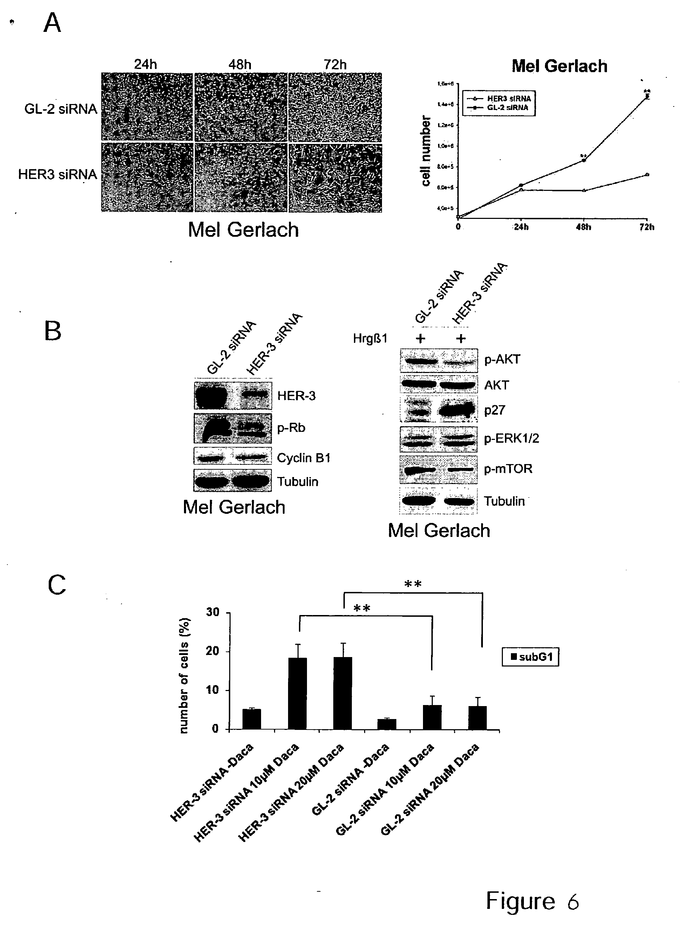

### Fig. 6 HER3 knock-down in Mel Gerlach melanoma cells.

**A** *HER3* knock-down inhibits proliferation of Mel Gerlach melanoma cells. *HER3* knock-down and *GL-2* control cells were counted at the indicated time points and data are shown as mean ± SDM. Pictures were taken on a Zeiss Axiovert 300 microscope.
**B.** *HER3* knock-down impairs AKT activity in Mel Gerlach melanoma cells. Western blots for HER3, p-AKT, AKT, p27, p-Rb, Cyclin B1, p-ERK1/2 and p-mTOR are shown. Tubulin served as loading control. **C** HER3 knock-down sensitizes Mel Gerlach melanoma cells to Dacarbazine-induced apoptosis. Mel Gerlach *HER3* knock-down and *GL-2* control cells were either treated with 10 or 20µM Dacarbazine or left untreated for 48 hours and apoptosis was analyzed by flow cytometry.

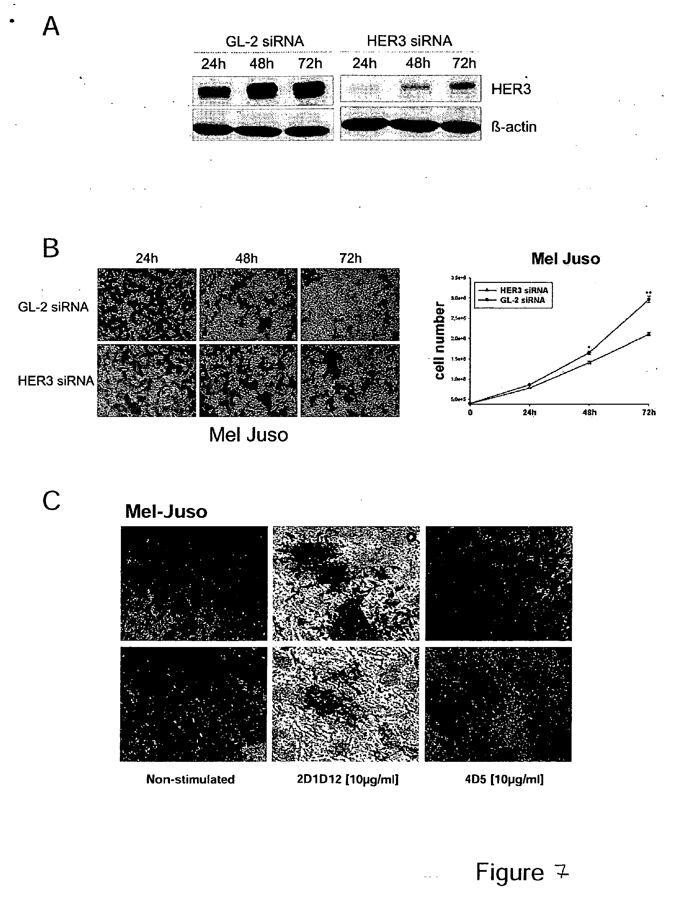

### Fig. 7 HER3 knock-down in Mel Juso melanoma cells

**A** *HER3* knock-down in Mel Juso melanoma cells. Cells were lysed at the indicated time points and subjected to Western blot analysis for HER3. β-actin served as a loading control. **B** *HER3* knock-down inhibits proliferation of Mel Juso melanoma cells. **C** An Anti-HER3 monoclonal antibody (cl. 2D1D12) inhibits the invasion of Mel Juso melanoma cells. Mel Juso cells were either incubated with an anti-HER3 monoclonal antibody (cl. 2D1D12; 10µg/ml), an anti-HER2 monoclonal antibody (4D5; 10µg/ml) or left untreated.

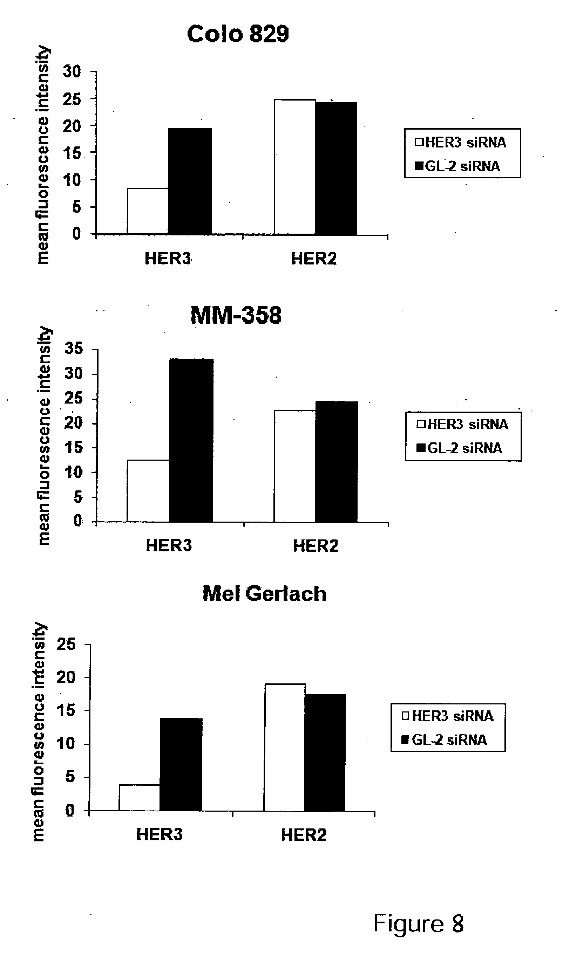

### Fig. 8 HER3 knock-down does not alter HER2 surface expression in melanoma cell lines

**A-C** HER3 and HER2 surface expression was measured by indirect flow cytometry. Colo 829, MM-358 and Mel Gerlach *HER3* knock-down cells were incubated with specific primary antibodies for HER2 and HER3 for 1 hour and afterwards with a PE-labelled secondary antibody followed by flow cytometry. The fluorescence intensities for HER3 and HER2 are shown.

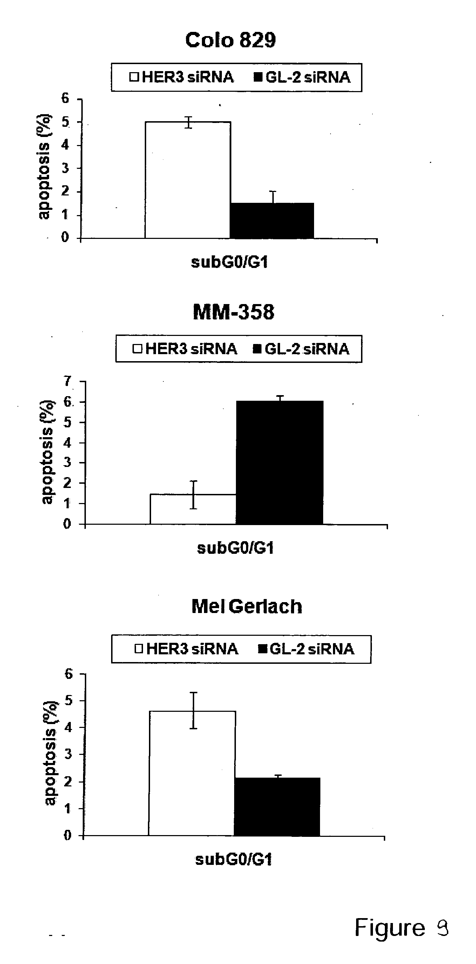

### Fig. 9 HER3 knock-down does not induce apoptosis in melanoma cells

200.000 cells were seeded in 6 well plates and transfected with HER3 or GL-2 siRNAs using olgofectamine (Invitrogen). Cells were trypsinized after 48 hours and analyzed by Propidium Iodide staining as described in Material and Methods. Apoptotic cells were identified as the subGO/G1 population and quantified using the Cell Quest Pro software (Beckton Dickinson Biosciences).

### Examples

### 1. Material and Methods

### 1.1 Melanoma Patients

Formalin-fixed, paraffin embedded tissue of 130 primary cutaneous melanoma and 87 metastases was immunohistochemically analyzed for HER3. The patient age ranged from 19 to 90 years. Clinical follow-up was available in all of the patients (mean clinical follow up was 56 ± 25 months). There were 60 nodular (NMM), 42 superficial spreading (SSM), 3 lentiginous (LMM), 9 acral lentiginous (ALM) and 16 not otherwise specified (NOS) melanoma. All melanoma had a Breslow tumor thickness between 0.4 and 17 mm. 53 of 130 patients (41%) had metastases during follow up and 24 of 130 patients (18%) died. Matched tumor samples of primary melanoma and metastases were available for 20 patients. 54 of the 130 patients with primary cutaneous melanomas were previously reported in a sentinel lymph node study (23). Approval was obtained from a local Institutional Ethical Committee and written informed consent signed by all study participants.

### 1.2 Tissue Micro array construction and Immunohistochemistry

A morphologically representative region of a paraffin "donor" blocks was chosen to prepare the melanoma tissue micro arrays. The representative region was taken with a core tissue biopsy (diameter: 0.6 mm; height: 3-4 mm) and precisely arrayed into a new "recipient" paraffin block using a customer built instrument (24). After the block construction was completed, 4.0 µm sections of the resulting tumor tissue micro array block were cut with a microtome and used for further analysis.

HER3 and Ki-67 immunohistochemistry was performed using a Ventana Benchmark automated staining system (Ventana Medical Systems, Tucson, Arizona). For antigen retrieval, slides were heated with cell conditioner 1 (standard procedure). Endogenous biotin was blocked with the appropriate kit. Primary antibodies against HER3 (Santa Cruz, clone C-17, dilution 1:50) and Ki-67 proliferation antigen (clone MIB-1, dilution 1:20 were applied and revealed with the iVIEW DAB detection kit, yielding a brown reaction product. The signal was enhanced with the Ventana amplification kit. Slides were counterstained with hematoxylin prior to glass cover slipping. The specificity of the staining was controlled by using iso-type antibody controls, secondary antibody controls or blocking peptides. The analysis of the tissue micro arrays was done using a Zeiss Axiovert 300 microscope.

### 1.3 Evaluation of HER3 expression

To determine the expression frequencies of HER3, a semi-quantitative scoring system was applied following the German Immunohistochemical scoring (GIS) system in which the final immuno-reactive score equaled the product of the percentage of positive cells times the average staining intensity. Percentage of positive cells was graded as follows: 0 = negative, 1 = up to 10% positive cells, 2 = 11 to 50%, 3 = 51 to 80%, 4 = >80%. Staining intensity of 0 = negative, 1 = weakly positive, 2 = moderately positive, 3 = strongly positive (25 - 27). The extent of KI-67 staining was recorded as the Ki-67 Labelling Index (number of marked nuclei per 100 melanoma cells). All stainings were evaluated by two different investigators.

### 1.4 Statistical analysis

The protein expression frequency for HER3 was analyzed by dividing the GIS score in three groups with GIS 1-4 = no/low, GIS 5-8 = moderate and GIS 9-12 = high expression. For statistical analysis, two groups were considered which were divided through GIS-dichotomization at the median (low: GIS ≤6 and high: GIS >6). Correlations between HER3 and Ki-67 were analyzed using Spearman's rank correlation. The overall survival of melanoma patients was estimated by the Kaplan Meier method and differences between groups were assessed by the log-rank test. The overall survival was defined as the time of primary tumor diagnosis to the last follow-up visit or patient death. All p-values were calculated using the two-sided Fisher's exact Test or the paired Student's T-Test and p-values < 0.05 were considered statistically significant. A multivariable Cox regression model was adjusted, testing the independent prognostic relevance of HER3 in melanoma patients. The following clinical variables were considered: age (≤ 60 years vs. > 60 years), sex (male vs female), tumor thickness (≤ 2mm vs > 2mm) and metastases during follow up. The proportionality assumption for all variables was assessed with log-negative-log survival distribution functions. For the analysis of matched tumor metastases pairs, the GIS values of HER3 in primary melanoma and melanoma metastases were directly compared and analyzed. The statistical analysis was performed with the SPSS 12.0 software (SPSS Inc., Chicago IL).

### 1.5 Cell culture and compounds

All cell lines used in this study were purchased from the American Type Culture Collection (ATCC) and cultured according to ATCC guidelines. Dacarbazine and Propidium-lodide were purchased from SIGMA. Heregulin β1 was purchased from R&D Systems and diluted in PBS prior to use.

### 1.6 RNA interference

*HER3* siRNAs were obtained from AMBION. Two independent siRNAs were used in all experiments. Sequences for *HER3* siRNAs were sense1 5'GGCUAUGUCCUCGUGGCCAtt 3', antisense1 5' UGGCCACGAGGACAUAGCCtg 3' and sense2 5' GGCAGUGUGUCCUGGGACUtt 3', antisense2 5' AGUCCCAGGACACACUGCCtg 3'. The *GL-2* siRNA (Dharmacon) was used as a negative control in all experiments. *GL-2*_sense 5' CGUACGCGGAAUACUUCGAtt 3', *GL-2*_antisense 5' UCGAAGUAUUCCGCGUACGtt 3'. Transfection of siRNAs was done using Oligofectamine (Invitrogen, CA) according to manufacturer's recommendation.

### 1.7 Antibodies, RT-PCR and Western Blot analysis

Antibodies against p-HER3 (Tyr 1289), p85, p-AKT (Ser473), CyclinB1, p-ERK1/2, pmTOR and p-Rb were all purchased from Cell Signaling (Beverly, MA). HRP conjugated rabbit secondary antibodies were from BioRad. Anti-Tubulin, Anti-β-actin and HRP-conjugated mouse secondary antibodies were from SIGMA. The anti-HER3 (clone 2F12) antibody was from Upstate and anti-HER3 (C-17) for immunohistochemistry as well as Akt 1/2 (H-136) were from Santa Cruz. Anti-p27 was purchased from Transduction Laboratories. Western blot analysis and immunoprecipitations were done as described previously (28). Total RNA was isolated using the RNeasy Mini Kit (Quiagen) and c-DNA was synthesized using the AMV Reverse Transcriptase (Roche) according to manufacturer's recommendations. RT-PCR primers for HER3: HER3_fwd 5' CTCCGCCCTCAGCCTACCAGTT 3' and HER3_rev 5' TGCTCCGGCTTCTACACATTGACA 3' (Tm=64°C) and for Tubulin: Tubulin_fwd; 5' AAGTGACAAGACCATTGGGGGAGG 3' and Tubulin_rev 5' GGGCATAGTTATTGGCAGCATC 3' (Tm = 55°C). All PCR reactions were done in an Eppendorf thermocycler (Eppendorf).

### 1.8 Proliferation Assay

75000 or 250000 cells were seeded in 24 well or 6 cm plates and transfected with *HER3* or *GL-2* siRNAs using oligofectamine (Invitrogen). The cells were grown in the presence of medium containing 10% FCS and counted (Coulter counter, Beckton Dickinson) at the indicated time points. The data are shown as mean ± SDM.

### 1.9 Migration and Invasion Assay

200.000 cells were seeded in 6 well plates and transfected with *HER3* or *GL-2* siRNAs using oligofectamine (Invitrogen). The cells were serum-starved for 24 hours and 25.000 cells were either seeded on to a membrane or on to a growth factor reduced matrigel coated membrane with 8 µM pores of a modified boyden chamber (Schubert and Weiss) containing 500µl serum-free medium. 10% fetal calf serum served as chemoattractant. The cells were allowed to migrate or invade for 20 or 24 hours, respectively. The cells were stained with crystal violet, washed in PBS and pictures were taken on a Zeiss Axiovert 300 microscope. For quantification cells in at least 10 random fields were counted and the data are shown as mean ± SDM.

### 1.10 HER3 blocking antibody - Migration and Invasion experiments

The HER3 blocking antibody (cl. 105.5) was purchased from Upstate, NY. The second HER3 blocking antibody (cl. 2D1D12) was generated in the Department of Molecular Biology at the Max-Planck Institute of Biochemistry.

The migration and invasion assays were performed as described previously (19, 29). Briefly, 300.000 cells were seeded in 6 cm plates and serum-starved in medium containing 0,1% FCS for 24 hours. 200.000 cells per ml were incubated with 10µg/ml HER3 blocking antibody for 1 hour and 50.000 cells were then seeded either on to a membrane or on to a growth factor reduced matrigel coated membrane with 8 µM pores of a modified boyden chamber (Schubert and Weiss) containing 500µl serum free medium. Conditioned NIH 3T3 medium containing 0.01% ascorbic acid and Heregulin β1 (100ng/ml) was used as a chemoattractant. Migrated or invaded cells were stained by crystal violet, washed in PBS and analyzed using a Zeiss Axiovert 300 microscope. For quantification at least 10 random fields were counted and the data are presented as mean ± SDM.

To asses the HER3 phosphorylation state, cells were serum-starved for 24 hours, incubated with 10µg/ml blocking antibody for 1 hour, stimulated with 50ng/ml Heregulin β1 for 2 hours, lysed and subjected to Immunoprecipitations using a specific HER3 antibody.

### 1.11 Apoptosis Assay (Propidium Iodide staining)

200.000 cells were seeded into 6 well plates (Nunc) and transfected with *HER3* or *GL-2* siRNAs. Apoptosis was induced by adding either 10 or 20µM Dacarbazine in DMSO to the medium. After 48 hours the supernatant of each reaction was collected and the cells were trypsinized. After centrifugation the cells were incubated for two hours in a Propidium-lodide buffer (0.1% Na-Citrate, 0.1% Triton X-100, 20µM Propidium-lodide) and thereafter subjected to flow cytometric analysis (Beckton-Dickinson Biosciences). Apoptotic cells were identified as the sub G0/G1 peak and quantified using the Cell Quest Pro software (Beckton Dickinson).

### 1.12 Indirect flow cytometry

The antibodies used for HER3 and HER2 were described elsewhere (18, 19). 500.000 cells were seeded in 10 cm dishes (Falcon) and transfected with HER3 or GL-2 siRNAs using oligofectamine (Invirtogen). Cells were collected after 24 hours using 10mM EDTA and dissolved in 1 ml 3% FCS in PBS. The cell number was adjusted to 250.000 cells per reaction and cells were incubated for 30 minutes with 10 µg/ml of each primary antibody at 4°C. The cells were washed 3 times in 3% FCS/PBS and incubated with a PE-labeled secondary (1:1000) antibody at 4°C for 30 minutes. After three more washes in 3% FCS/PBS the fluorescence intensity was measured in a flow cytometer (Beckton Dickinson Biosciences) and analyzed using the Cell Quest Pro software.

### 2. Results

### 2.1 HER3 is frequently expressed in primary melanoma and metastases

HER3 protein expression was investigated in 130 primary malignant melanoma and 87 metastases using tissue micro arrays. HER3 immuno reactivity was accentuated at the cell membrane. In primary melanoma, moderate to high HER3 expression levels were found in 85 of 130 cases (65%) (Fig. 1A-C and 1E). Furthermore, HER3 was highly expressed in 35 of 87 melanoma metastases (40%) (Fig. 1D and 1E). Importantly, HER3 expression was undetectable in primary melanocytes (Fig. 1 F). Interestingly, moderate to high HER3 expression significantly correlated with increased tumor cell proliferation (Ki-67 Labeling Index) in primary malignant melanoma (p=0,008; data not shown).

### 2.2 HER3 expression in melanoma progression

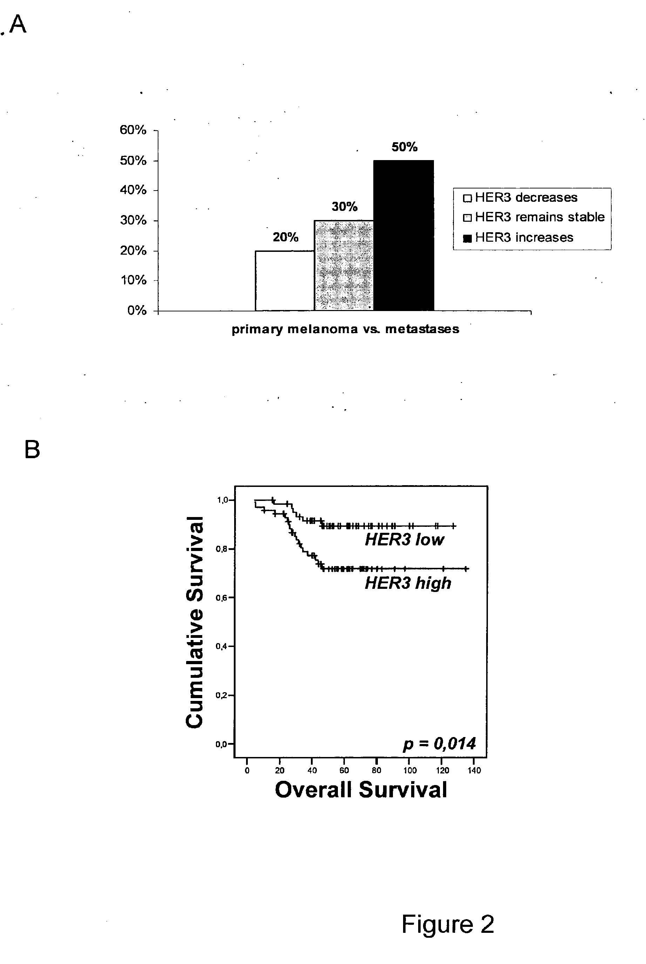

Matched tumor samples of primary melanoma and metastases were studied in 20 patients. Remarkably, 10 of 20 (50%) patients showed an increase of HER3 expression in the metastases compared to the primary tumor. Six of 20 (30%) matched tumor samples showed a similar expression of HER3 and only 4 of 20 (20%) patients showed less HER3 expression in the metastases when compared to the primary tumor (Fig. 2A).These results show that in a majority of cases HER3 expression remains either stable or even increases during disease progression.

Importantly, Kaplan-Meier analysis showed that HER3 expression was significantly associated with tumor specific survival (p=0,014) (Fig. 2B). In a multivariate analysis, an adjusted Cox regression model was developed for the assessment of the overall survival rate. The characteristics of the variables are shown in Table 1.

**Table 1**

| **Table 1.** Multivariate analysis of factors possibly influencing overall survival (forward LR method) | | | |
|---|---|---|---|
| **Variable** | **Characteristics** | **Hazard ratio** **(95% Cl)** | ***p*** |
| Age | 0 ≤ 60 years 1 > 60 years | - | NS |
| Sex | 0 = male 1 = female | - | NS |
| Tumor thickness | 0 ≤ 2 mm 1 > 2 mm | - | NS |
| HER-3 | 0 low 1 high | 2.6 (1.04-6.6) | **0.041^{a}** |
| Metastases during follow-up | 0 no metastases 1 metastases | 22.2 (5.2-95.5) | **0,000** |

| | | | |
|---|---|---|---|
| ^{a} Bold type representing data with p < 0.05 Abbreviations: NS, not significant; HR, hazard ratio; 95% Cl, 95% confidence interval | | | |

The clinical variables used in the analysis were age, sex, metastases during progression, tumor thickness and HER3 expression (HER3 Immunohistochemistry score). In this model, metastases (p=0,000) and HER3 expression (p=0.041) were correlated with poor prognosis. the hazard ratio for death from melanoma concerning HER3 status was 2.6 (95% Confidence Interval: 1,042-6,671); accordingly, in cases with high HER3 expression, the probability of tumor-related death was almost three times higher than in cases with low HER3 staining. Because of the assumption of proportional hazards, the probability of melanoma-related dearth was consistently valid during the entire observation period. Taken together, these results indicate that HER3 is a critical parameter for melanoma prognosis and progression.

### 2.3 HER3 knock-down inhibits melanoma cell proliferation

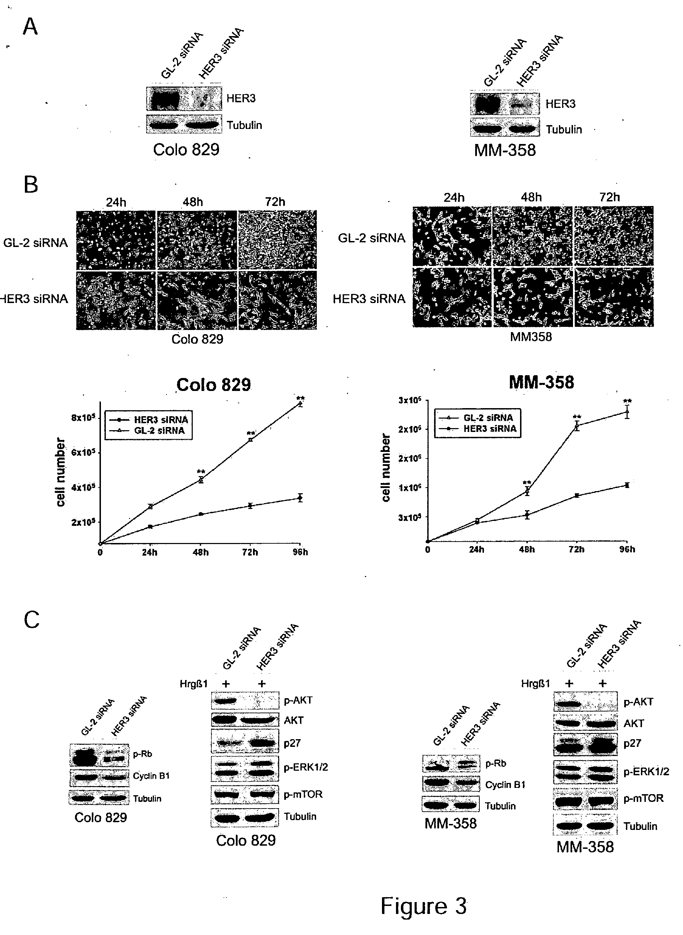

In order to further characterize the role of HER3 in melanoma, *HER3* was down-regulated by specific siRNAs in Colo 829, MM-358, Mel Gerlach and Mel Juso human melanoma cell lines (Fig. 3A, Fig. 6A and 7A). Strikingly, depletion of *HER3* strongly inhibited proliferation in these cell lines (Fig. 3B, 6A and 7B). Importantly, HER3 predominantly signals via the P13K-AKT pathway in the regulation of cell proliferation and -survival. Indeed, AKT activation was impaired in heregulin β1 stimulated knock-down cell lines indicating that HER3 signals via the PI3K-AKT pathway in melanoma cells (Fig. 3C and Fig. 6B). In contrast, p-ERK and pmTOR levels remained unchanged (Fig. 3C and Fig. 6B). In addition, an up-regulation of p27 protein levels was observed which is most likely a direct consequence of the impaired AKT activity (Fig. 3C and Fig. 6B) since AKT is known to trigger p27 degradation and cell cycle progression (30 - 33). To further characterize the mechanism of the growth inhibition, proteins implicated in cell cycle control were analyzed. As shown in Fig. 3C, *HER3* knock-down led to reduced Cyclin B1 levels and decreased Rb phosphorylation. Importantly, HER2 protein levels were not altered in *HER3* down-regulated cells suggesting that the effects are specific for HER3 (Fig. 8). The obtained data demonstrate that HER3 signals via the PI3K-AKTp27 pathway in melanoma cells and thereby appears to promote melanoma cell proliferation.

### 2.4 HER3 knock-down inhibits melanoma cell migration and invasion

Melanoma metastases frequently express high levels of HER3 (Fig. 1D and 1E). Given the association between HER3 expression and poor survival, one might hypothesize that HER3 plays a role in melanoma progression. Increased tumor cell migration and invasion are important prerequisites for metastasis. In order to address this question, the migration and invasion of Colo 829, Mel Gerlach and MM-358 melanoma cells upon interference with *HER3* expression were analyzed. As shown in Figure 4A, *HER3* knock-down efficiently blocked melanoma cell migration in all three cell lines. In invasion experiments Colo 829 and Mel Gerlach cells were markedly inhibited upon *HER3* suppression (Fig. 4B) while MM-358 cells did not invade the matrix even in untransfected controls (data not shown). These results establish HER3 as a potent mediator of melanoma cell migration and invasion.

### 2.5 HER3 knock-down sensitizes melanoma cells to Dacarbazine-induced apoptosis

In contrast to previously published data in lung cancer cells which undergo apoptosis in the absence of *HER3* (15), suppression of *HER3* and subsequent down-regulation of AKT activity did not induce apoptosis in melanoma cells (Fig. 9). Nevertheless, it reasoned that inhibition of *HER3* might synergize with chemotherapy in the induction of apoptosis in melanoma cells. To date, Dacarbazine is the only FDA-approved drug for melanoma therapy (4). Indeed, it was found that Dacarbazine-induced apoptosis was significantly increased in *HER3* knock-down melanoma cells (Fig. 4C and Fig. 6C). These results indicate that combination therapy with HER3 and Dacarbazine-like drugs might be useful for the treatment of malignant melanoma.

### 2.6 Anti-HER3 monoclonal antibodies block Heregulin-induced HER3 activation and melanoma cell migration and invasion

It has been shown above that HER3 is frequently overexpressed in primary melanoma and melanoma metastases and that high HER3 levels confer poor prognosis for melanoma patients. In addition, the RNA interference experiments shown above suggest that HER3 may be a potential target for melanoma therapy. To test this hypothesis *in vitro* Colo 829, MM-358, Mel Gerlach and Mel Juso melanoma cells were treated with anti-HER3 monoclonal antibodies. Remarkably, Heregulin induced activation of HER3 and its association with the PI3-K subunit p85 was completely abrogated in antibody-treated cells when compared to controls (Fig. 5A). In addition, anti-HER3 monoclonal antibodies cause receptor degradation or internalization similar to previously obtained results in breast cancer cells (19). Importantly, Anti-HER3 monoclonal antibodies are able to block Heregulin-induced migration and invasion of human melanoma cell lines (Fig. 5B, 5C and Fig. 7C) indicating that such antibodies may be effective anti-melanoma therapeutics. Taken together, these results indicate that targeting HER3 is a promising new opportunity for melanoma therapy.

### 3. Discussion

According to the present invention, HER3 expression in melanoma and its significant association with proliferation are shown. Furthermore, frequent and high HER3 expression in melanoma metastases compared to primary melanoma is demonstrated, indicating that HER3 may be involved in disease progression. In addition, high levels of HER3 significantly correlated with decreased life expectancy of patients establishing HER3 as a novel prognostic marker for melanoma. Importantly, HER3 expression is undetectable in primary melanocytes suggesting that HER3 overexpression specifically occurs during melanoma development. To address the role of HER3 in melanoma development and progression, human melanoma cell lines upon siRNA interference with *HER3* expression were analyzed. Reduction of *HER3* expression resulted in reduced cell proliferation, migration and invasion. On the molecular signaling level, suppression of *HER3* led to reduced AKT activity and increased p27 protein levels which may be the cause of the observed growth inhibition. Notably, *HER3* ablation did not affect the ERK1/2 and mTOR kinases suggesting that inhibition of other downstream pathways such as PI3K-AKT seems to be sufficient to block melanoma cell proliferation, migration and invasion. To further analyze whether HER3 may qualify as a novel target in melanoma therapy, human melanoma cells lines were treated with anti-HER3 monoclonal antibodies. It was found that such antibodies can inhibit heregulin-induced HER3 phosphorylation leading to receptor internalization or degradation. Furthermore, it is shown that binding of p85, the regulatory subunit of PI3K, to HER3 is abrogated upon antibody incubation demonstrating that signaling via the PI3K/AKT signaling pathway is inhibited in these cells. Importantly, melanoma cell migration and invasion are greatly reduced in antibody-treated cells when compared to controls. These data demonstrate that anti-HER3 antibodies can inhibit HER3 signaling via the PI3K pathway in melanoma cell lines and thereby seem to block melanoma cell migration and invasion. Importantly, it was shown in Mel Juso melanoma cells that a monoclonal antibody specific for HER2 did not affect melanoma cell invasion suggesting that inhibition of HER3 alone is sufficient to block melanoma invasiveness (Fig. 7C).

It was shown that the PI3K-AKT signaling pathway has essential functions in the regulation of cell survival and it is often found up-regulated in human cancer cells (35). Although interference with HER3 function and the consequential down-regulation of AKT were not sufficient to induce major apoptosis of melanoma cells, it was tested whether a combination of HER3 down-regulation with chemotherapeutic drug treatment would increase cell death. HER3 knock-down cells were highly sensitive to apoptosis induced by the FDA-approved drug Dacarbazine suggesting that a combination with agents interfering with HER3 might prove effective in the treatment of malignant melanoma.

Taken together, our results establish HER3 as a target for melanoma therapy.

### References

1. Miller AJ, Mihm MC Jr. Melanoma N Engl J Med 2006;355;51-65.
2. Chin L, Garraway LA, Fisher DE. Malignant melanoma: genetics and therapeutics in the genomic era. Genes Dev 2006;20;2149-2182.
3. Fecher LA, Cummings SD, Keefe MJ, et al. Toward a molecular classification of melanoma. J Clin Oncol 2007; 25;1606-1620.
4. Chudnovsky Y, Khavari PA, Adams AE. Melanoma genetics and the development of rational therapeutics. J Clin Invest 2005;115;813-824.
5. Gschwind A, Fischer OM, Ullrich A. The discovery of receptor tyrosine kinases: targets for cancer therapy. Nat Rev Cancer 2004;4;361-370.
6. Yarden Y, and Sliwkowski MX. Untangling the ErbB signalling network. Nat Rev Mol Cell Biol 2001;2;127-137.
7. Hynes NE, Lane HA. ERBB receptors and cancer: the complexity of targeted inhibitors. Nat Rev Cancer 2005;5;341-354.
8. Tanner B, Hasenclever D, Stern K, et al. ErbB-3 predicts survival in ovarian cancer. J Clin Oncol 2006;24;4317-4323.
9. Witton, CJ, Reeves JR, Going JJ, et al. Expression of the HER1-4 family of receptor tyrosine kinases in breast cancer. J Pathol 2003;200;290-297.
10. Yi ES, Harclerode D, Gondo M, et al. High c-erbB-3 protein expression is associated with shorter survival in advanced non-small cell lung carcinomas. Mod Pathol 1997;10;142-148.
11. Sliwkowski MX, Schaefer G, Akita RW, et al. Coexpression of erbB2 and erbB3 proteins reconstitutes a high affinity receptor for heregulin. J Biol Chem, 1994;14661-14665.
12. Carraway KL 3rd, Sliwkowski MX, Akita R, et al. The erbB3 gene product is a receptor for heregulin. J Biol Chem 1994;269;14303-14306.
13. Wallasch C, Weiss FU, Niederfellner G, et al. Heregulin-dependent regulation of HER2/neu oncogenic signaling by heterodimerization with HER3. Embo J 1995;14;4267-4275.
14. Schulze WX, Deng L, Mann M. Phosphotyrosine interactome of the ErbBreceptor kinase family. Mol Syst Biol 2005;1;2005 0008.
15. Sithanandam G, Fornwald LW, Fields J, et al. Inactivation of ErbB3 by siRNA promotes apoptosis and attenuates growth and invasiveness of human lung adenocarcinoma cell line A549. Oncogene 2005;24;1847-1859.
16. Guy PM, Platko JV, Cantley LC, Cerione RA, Carraway KL 3rd. Insect cellexpressed p180erbB3 possesses an impaired tyrosine kinase activity. Proc Natl Acad Sci U S A 1994; 91;8132-8136.
17. Holbro T, Beerli RR, Maurer F, Koziczak M, Barbas CF 3rd, and Hynes NE. The ErbB2/ErbB3 heterodimer functions as an oncogenic unit: ErbB2 requires ErbB3 to drive breast tumor cell proliferation. Proc Natl Acad Sci U S A 2003; 100;8933-8938.
18. van der Horst EH, Weber I, Ullrich A. Tyrosine phosphorylation of PYK2 mediates heregulin-induced glioma invasion: novel heregulin/HER3-stimulated signaling pathway in glioma. Int J Cancer 2005; 113;689-698.
19. van der Horst EH, Murgia M, Treder M, Ullrich A. Anti-HER3 MAbs inhibit HER3-mediated signaling in breast cancer cell lines resistant to anti-HER2 antibodies. Int J Cancer 2005;115;519-527.
20. Sergina NV, Rausch M, Wang D, et al. Escape from HER-family tyrosine kinase inhibitor therapy by the kinase-inactive HER3. Nature 2007;445;437-441.
21. Liu B, Ordonez-Ercan D, Fan Z, et al. Downregulation of erbB3 abrogates erbB2-mediated tamoxifen resistance in breast cancer cells. Int J Cancer 2007;120;1874-1882.
22. Engelman JA, Zejnullahu K, Mitsudomi T, et al. MET amplification leads to gefitinib resistance in lung cancer by activating ERBB3 signaling. Science 2007;18;1039-43.
23. Mihic-Probst D, Mnich CD, Oberholzer PA, et al. p16 expression in primary malignant melanoma is associated with prognosis and lymph node status. Int J Cancer 2006;118;2262-8.
24. Kononen J, Bubendorf L, Kallioniemi A, et al. Tissue microarrays for highthroughput molecular profiling of tumor specimens. Nat Med 1998;4;844-847.
25. Shim V, Gauthier ML, Sudilovsky D, et al. Cyclooxygenase-2 expression is related to nuclear grade in ductal carcinoma in situ and is increased in its normal adjacent epithelium. Cancer Res 2003;63;2347-2350.
26. Khoury T, Tan D, Wang J, et al. Inclusion of MUC1 (Ma695) in a panel of immunohistochemical markers is useful for distinguishing between endocervical and endometrial mucinous adenocarcinoma. BMC Clin Pathol 2006;6;1.
27. Hutterer M, Knyazev P, Abate A, et al., Axl and Growth Arrest- Specific Gene 6 are frequently overexpressed in human gliomas and predict poor prognosis in patients with glioblastoma multiforme. Clin Cancer Res 2008;14;1.
28. Hackel PO, Gishizky M, Ullrich A. Mig-6 is a negative regulator of the epidermal growth factor receptor signal. Biol Chem 2001;382;1649-1662.
29. Albini A, Iwamoto Y, Kleinman HK, Martin GR, Aaronson SA, Kozlowski JM, McEwan RN. A rapid in vitro assay for quantitating the invasive potential of tumor cells. Cancer Res. 1987 Jun 15;47(12):3239-45.
30. Viglietto G, Motti ML, Bruni P, et al. Cytoplasmic relocalization and inhibition of the cyclin-dependent kinase inhibitor p27(Kip1) by PKB/Akt-mediated phosphorylation in breast cancer. Nat Med 2002;8;1136-1144.
31. Shin I, Yakes FM, Rojo F, et al. PKB/Akt mediates cell-cycle progression by phosphorylation of p27(Kip1) at threonine 157 and modulation of its cellular localization. Nat Med 2002;8;1145-1152.
32. Liang J, Zubovitz J, Petrocelli T, et al. PKB/Akt phosphorylates p27, impairs nuclear import of p27 and opposes p27-mediated G1 arrest. Nat Med 2002;8;1153- 1160.
33. Liang J, Slingerland JM. Multiple roles of the PI3K/PKB (Akt) pathway in cell cycle progression. Cell Cycle 2003;2;339-345.
34. Gray-Schopfer V, Wellbrock C, Marais R. Melanoma biology and new targeted therapy. Nature 2007;445;851-857.
35. Vivanco I, Sawyers CL. The phosphatidylinositol 3-Kinase AKT pathway in human cancer. Nat Rev Cancer 2002;2;489-501.

## Claims

1. Inhibitor of HER3 for the treatment of melanoma, preferably refractory melanoma.

2. The inhibitor of HER3 according to claim 1,
which is an inhibitor of HER3 expression, which preferably acts by down-regulation of HER3.

3. The inhibitor of HER3 according to claim 1 or 2,
wherein is an inhibitor of Heregulin-induced HER3 activation.

4. The inhibitor of HER3 according to any of the claims 1 to 3,
which is an inhibitor of melanoma cell proliferation, migration and/or invasion.

5. The inhibitor of HER3 according to any of the claims 1 to 4,
wherein the inhibitor is selected from the group consisting of nucleic acids, in particular siRNA, antisense oligonucleotides or ribozymes, peptidic compounds, in particular antibodies or antibody fragments and small organic molecules and combinations thereof.

6. The inhibitor of HER3 according to any of the claims 1 to 5,
wherein the melanoma is primary melanoma or metastases.

7. The inhibitor of HER3 according to any of the claims 1 to 6,
wherein the melanoma is selected from the group consisting of malignant melanoma, clear cell sarcoma, mucosal melanoma and/or uveal melanoma.

8. The inhibitor of HER3 according to any of the claims 1 to 7,
which is an enhancer of apoptosis.

9. The inhibitor of HER3 according to any of the claims 1 to 8,
which sensitizes the melanoma for treatment with a further agent, preferably a chemotherapeutic agent or radiation therapy.

10. Pharmaceutical composition comprising an inhibitor of HER3 according to any of the claims 1 to 9.

11. The pharmaceutical composition according to claim 10,
which is to be administered in combination with further chemotherapeutic and/or immunotherapeutic agents and/or before, during and/or after surgical removal of the melanoma and/or radiation therapy.

12. The pharmaceutical composition according to claim 10 or 11,
wherein the further agent is Dacarbazine, Herceptin, Omnitarg, Lapatinib and/or Temozolomide.

13. The pharmaceutical composition according to any of the claims 10 to 12 further comprising at least one further chemotherapeutic and/or immunotherapeutic agent, preferably Dacarbazine, Herceptin, Omnitarg, Lapatinib and/or Temozolomide.

14. In vitro method for the diagnosis and/or prognosis of melanoma comprising
(a) detecting HER3 levels in cells to be analysed and
(b) correlating the detected HER3 levels with proliferation, tumor progression and/or patient survival.

15. The in vitro method according to claim 14,
wherein up-regulated HER3 levels indicate melanoma.
